Europäisches Patentamt

European Patent Office

Office européen des brevets

(19)

(11) Veröffentlichungsnummer : **0 072 439**
**B1**

(12) **EUROPÄISCHE PATENTSCHRIFT**

(45) Veröffentlichungstag der Patentschrift :
05.03.86

(21) Anmeldenummer : 82106390.6

(22) Anmeldetag : 16.07.82

(51) Int. Cl.⁴ : **B 01 J 23/84**, B 01 J 23/86,
B 01 J 23/88, B 01 J 27/18,
C 07 C  5/333, C 07 C 15/46

(54) **Dehydrierungskatalysator, Verfahren zur Herstellung desselben und Verwendung zur Dehydrierung von Ethylbenzol zu Styrol.**

(30) Priorität : 13.08.81 DE 3132014

(43) Veröffentlichungstag der Anmeldung :
23.02.83 Patentblatt 83/08

(45) Bekanntmachung des Hinweises auf die Patenterteilung : 05.03.86 Patentblatt 86/10

(84) Benannte Vertragsstaaten :
BE DE FR GB NL

(56) Entgegenhaltungen :
DE-A- 1 542 328
FR-A- 2 387 199
GB-A-   667 876
GB-A- 2 091 757
US-A- 2 495 278
US-A- 3 100 234
US-A- 3 409 688

(73) Patentinhaber : **BASF Aktiengesellschaft**
**Carl-Bosch-Strasse 38**
**D-6700 Ludwigshafen (DE)**

(72) Erfinder : **Mross, Wolf Dieter, Dr.**
**Anselm-Feuerbach-Strasse 21**
**D-6710 Frankenthal (DE)**
Erfinder : **Biedenkapp, Dieter, Dr.**
**Froebelstrasse 26**
**D-6700 Ludwigshafen (DE)**
Erfinder : **Schwarzmann, Matthias, Dr.**
**Carl-Bosch-Strasse 54**
**D-6703 Limburgerhof (DE)**
Erfinder : **Schweter, Walter, Dr.**
**Am Weidenschlag 110**
**D-6700 Ludwigshafen (DE)**

0 072 439

**Beschreibung**

Die vorliegende Erfindung betrifft einen Dehydrierungskatalysator, ein Verfahren zu dessen Herstellung und seine Verwendung zur Dehydrierung von Ethylbenzol zu Styrol.

Zum Stand der Technik nennen wir :

(1) Kunststoff-Handbuch, Band V, Polystyrol, Carl Hanser-Verlag, Seiten 39 bis 47, (1969)

(2) DE-A-2 406 280

(3) US-A-4 144 197 und

(4) DE-A-2 544 185

(5) US-A-3 409 688

(6) US-A-3 100 234

Die katalytische Dehydrierung von Ethylbenzol zu Styrol ist ein technisch in großem Umfang ausgeübtes Verfahren. Dabei wird Ethylbenzol zusammen mit Wasserdampf bei Temperaturen zwischen 500 und 700 °C über ein Katalysatorbett geleitet. Das Verfahren kann adiabatisch oder isotherm durchgeführt werden. Beim adiabatischen Verfahren wird Wärme für die Ethylbenzol-Dehydrierung durch überhitzten Wasserdampf dem Reaktionsgemisch zugeführt. Bei dem isotherm durchgeführten Verfahren wird die Wärme für die endotherme Reaktion mit Hilfe von Wälzgas auf die Rohre eines Rohrbündelreaktors übertragen. Eine ausführliche Beschreibung dieser beiden technisch vorwiegend angewendeten Verfahren findet sich in (1). In (1) ist auch eine Vielzahl von geeigneten Dehydrierungskatalysatoren beschrieben, die im wesentlichen aus Gemischen verschiedener Metalloxide bestehen. Die technisch wichtigsten Katalysatoren sind dabei auf Basis von Eisenoxid aufgebaut und enthalten entweder Chromoxid oder Aluminiumoxid als Stabilisatoren sowie Kaliumoxid. Außerdem enthalten heute gebräuchliche Katalysatoren als Promotoren Oxide von Metallen der 5. oder 6. Nebengruppe, insbesondere werden die Oxide des Vanadins und die von Molybdän und Wolfram angewendet. Gelegentlich finden auch Oxide der 5. Hauptgruppe, insbesondere des Phosphors, Verwendung.

Aus (5) sind Dehydrierungskatalysatoren mit erhöhter Selektivität für Kohlenwasserstoffe, insbesondere zur Herstellung von Styrol, auf der Basis von Eisenoxid bekannt, die als Promotor auch ein Alkalimetalloxid oder Alkalimetalloxidgemische enthalten können. Als bevorzugter Promotor wird Kaliumoxid alleine angegeben. Ebenfalls zur Erhöhung der Selektivität wird in (6) ein Dehydrierungskatalysator für Kohlenwasserstoffe, insbesondere zur Herstellung von Styrol, auf Eisenoxid-Basis vorgeschlagen, bei dem Mischungen von Kaliumverbindungen mit Verbindungen von Metallen aus der Gruppe Natrium, Lithium, Barium, Magnesium und Calcium zu einer Maximalausbeute an Styrol führen.

Da die bekannten Katalysatoren bei Temperaturen oberhalb 600 °C und bei hohen Wasserdampfpartialdrucken angewendet werden, beobachtet man besonders bei sehr selektiven Katalysatoren, daß das Kalium ausgetragen wird und sich als Kaliumcarbonat am Reaktorausgang abscheidet. Dieser Austrag des Kaliums wird erstaunlicherweise besonders bei niedrigen Wasserdampf- zu Ethylbenzolverhältnissen beobachtet, wie sie beim isothermen Verfahren angewendet werden.

Es bestand daher die Aufgabe, einen Katalysator vorzuschlagen, bei dem der Kaliumaustrag weitgehend unterbunden wird, und damit auch die Desaktivierung des Katalysators vermieden wird.

Die Lösung der Aufgabe gelingt durch Einhaltung der im Patentanspruch 1 genannten Maßnahmen.

Der erfindungsgemäße Dehydrierungskatalysator enthält die nachstehend genannten Bestandteile.

Diese Bestandteile sind jeweils in Gewichtsprozent angegeben und bezogen auf den nach der Trocknung bei 700 bis 1 000 °C calcinierten gebrauchsfertigen Katalysator (Komponenten a) bis e) inclusive der Lithiumverbindung).

Die Komponente a) repräsentiert mindestens eine Eisenverbindung, die in einem Anteil von 50 bis 95 Gew.%, berechnet als $Fe_2O_3$, im Katalysator zugegen ist. Als Eisenverbindungen zur Herstellung des Katalysators kann von den verschiedenen Eisenoxiden bzw. Eisenoxidhydraten ausgegangen werden. Erwähnt seien die bekannten rotbraunen oder gelben bzw. schwarzen Eisenoxidpigmente. Die Herstellung derartiger Ausgangsmaterialien ist bekannt.

Als Komponente b) enthält der erfindungsgemäße Katalysator 3 bis 50 Gew.%, vorzugsweise 5 bis 45 Gew.% mindestens einer Kaliumverbindung, berechnet als $K_2O$. Die Art der angewendeten Kaliumverbindungen richtet sich nach der Art der herstellung. Es werden vorzugsweise solche Verbindungen gewählt, die beim Calcinieren des Katalysators sich zum Teil zu $K_2O$ zersetzen ; genannt seien das Hydroxid, das Carbonat, das Bicarbonat, das Carboxilat, das Borat oder die Phosphate des Kaliums. Bevorzugt ist es, das Kalium als Kalilauge zur Unterstützung des Knetvorganges bei der Herstellung des Kontaktes in die Mischung der übrigen Katalysatorbestandteile einzubringen.

Als Komponente c) des erfindungsgemäßen Katalystors wird entweder eine Chrom- oder eine Aluminiumverbindung in einem Anteil von 0,1 bis 20 Gew.%, berechnet als $Cr_2O_3$ bzw. $Al_2O_3$, angewendet. Diese Komponente des Katalysators wirkt als Strukturstabilisator und verlängert die Standzeit der Katalysatormischung. Chrom wird gelegentlich wegen seiner höheren Toxizität nicht verwendet, das Aluminiumoxid scheint gegenüber dem Chrom jedoch weniger wasserdampfresistent zu sein. Bevorzugt werden von diesem Strukturstabilisator 0,1 bis 5 Gew.% angewendet.

Als Komponente d) des erfindungsgemäßen Katalysators wird mindestens eine Verbindung eines Elements der 6. Nebengruppe des Periodensystems, berechnet als $MeO_3$, angewendet. Besonders

2

bevorzugt wird diese Verbindung in einer Menge von 0,2 bis 15 Gew.%. Bevorzugt werden Verbindungen der Elemente Molybdän und Wolfram, in Mengen von 0,1 bis 15 % bei Molybdän, berechnet als $MoO_3$, und 0,2 bis 20 Gew.%, insbesondere 0,3 bis 12 Gew.% bei Wolfram, berechnet als $WO_3$, anwendet. Als Verbindungen werden zweckmäßigerweise die Sauerstoffsäuren bzw. deren Ammoniumsalze oder Kaliumsalze in der Oxidationsstufe + 6 für die Herstellung des Katalysators verwendet, da sich diese Verbindungen beim Calcinieren leicht zersetzen. Besonders bevorzugt werden vom Molybdän und Wolfram die sogenannten Heteropolysäuren mit der Phosphorsäure angewendet, z. B. die Wolframatophosphorsäure bzw. die Molybdatophosphorsäure.

Anstelle der Komponente d) oder zusammen mit dieser kann der erfindungsgemäße Katalysator 0,1 bis 20 Gew.%, mindestens eine Verbindung des Vanadins oder des Phosphors, berechnet als $V_2O_5$ bzw. $P_2O_5$ aufweisen (Komponente e). Bevorzugt werden von diesen Verbindungen Mengen von 0,1 bis 15 Gew.%, insbesondere 0,5 bis 10 Gew.%. Bei diesen Angaben sind die ggf. durch die Komponente d) eingebrachten Mengen an Phosphorsäure zu berücksichtigen. Als Verbindungen des Vanadins kommen insbesondere die Oxide, die Ammoniumsalze oder die Kaliumsalze der höchsten Oxidationsstufe in Betracht, z. B. $V_2O_5$, $NH_4VO_3$ bzw. $KVO_3$. Falls sowohl eine Verbindung des Vanadins als auch des Phosphors verwendet wird, werden bevorzugt die Heteropolysäuren des Vanadins mit Phosphorsäure, z. B. die Vanadatophosphorsäure oder ihr Ammonium- oder Kaliumsalz ausgewählt. Bei der Wahl der Kalisalze dieser Heteropolysäuren sind die entsprechenden Abschläge für die Komponente b) zu berücksichtigen. Falls Phosphor alleine verwendet wird, wird dieser in Form des Oxids, der Säure bzw. der Ammoniumsalze oder der Kaliumsalze angewendet oder gemeinsam mit der Komponente d) als Heteropolysäure eingebracht.

Wesentlich für den erfindungsgemäßen Dehydrierungskatalysator ist, daß er zusätzlich zu der Komponente e) einen Gehalt von 0,1 bis 5 Gew.%, insbesondere von 0,1 bis 1 Gew.% einer Lithiumverbindung, berechnet als $Li_2O$, aufweist. Zur Herstellung des Katalysators können Salze des Lithiums, die in Wasser schwer löslich sind, z. B. das Carbonat oder Phosphat, angewendet werden.

Der erfindungsgemäße Katalysator kann ggf. weitere als Promotoren wirkende Zusätze f) in üblichen Mengen enthalten. Beispielsweise seien genannt : Cobalt, Magnesium, Uran, Zink, Silber, Calcium, Strontium, Barium, Blei und die Seltenen Erdmetalle.

Die Herstellung des erfindungsgemäßen Katalysators wird in der Regel ausgehend von einer Mischung der Komponenten a) bis e) sowie ggf. weiterer Zusätze und einer Lithiumverbindung erfolgen. Diese Mischung wird in der Regel mit Wasser, ggf. unter Zusatz von Kalilauge sowie unter Zusatz von Verformungshilfsmitteln, wie Stärke, Methylcellulose oder Graphit als einem die Porosität verbessernden Mittel, verformt oder zunächst geknetet und anschließend verformt ; bevorzugt ist dabei die Knetung und die anschließende Verformung, insbesondere das Extrudieren. Die bei der Extrusion erhaltenen Formlinge werden zunächst bei Temperaturen im Bereich von 80 bis 300 °C getrocknet und danach bei Temperaturen im Bereich von 700 bis 1 000 °C calciniert.

Der nach dem Calcinieren erhaltene Katalysator weist vorzugsweise eine BET-Oberfläche unter 10 $m^2/g$, insbesondere eine solche von 5 $m^2/g$ auf. (Die BET-Oberfläche wird gemessen nach Brunauer, Emett, Teller, J. Amer. Chem. Soc. 60 (1938), Seite 309.)

Durch den Calcinierungsprozeß erreichen die Formlinge in der Regel auch eine ausreichende Härte. Zur Verbesserung der Porosität können, wie vorstehend bereits angedeutet, Verformungs- bzw. die Porosität verbessernde Mittel bei der Herstellung des Katalysators verwendet werden. Die Formlinge sollen möglichst so gestaltet werden, daß die Porennutzung nahezu 100 % beträgt. Besonders bevorzugt sind Strangpreßlinge in Form von zylindrischen Ringen oder sternförmige Strangpreßlinge wie sie in (4) beschrieben sind.

Die in den Beispielen und Vergleichsversuchen genannten Parameter wurden wie folgt bestimmt :

1. Aktivität

Als Aktivität ist die Temperatur in °C angegeben, bei der die organische Phase im Austrag des Reaktors 50 Gew.% Styrol enthält.

2. Selektivität

Das Abgas und die organische Phase werden nach einer Betriebszeit von 7 Tagen analysiert, und aus den Analysen wird die Selektivität berechnet.

3. Alkaliaustrag

Nach Beendigung des Versuches wird der Katalysator schichtweise ausgebaut. Als Maß für den Alkaliaustrag in Prozent wird die Abnahme des Kaliumgehaltes im ersten Drittel des Katalysatorbettes verwendet.

Die Erfindung wird nachstehend anhand von Beispielen und Vergleichsversuchen näher erläutert. Alle darin angegebenen Teile und Prozente beziehen sich, sofern nichts anderes vermerkt ist, auf das Gewicht.

Beispiele 1-14 und Vergleichsversuch

Zur Durchführung der Beispiele und des Vergleichsversuches wurden zunächst die Katalysatoren 1 bis 15 aus den in der Tabelle 1 bzw. 2 genannten Komponenten und Mengen hergestellt. Bei den Katalysatoren 1 und 2 wurde von Eisenrotpigment, bei den Katalysatoren 3 bis 14 von gelbem Eisenoxidhydrat ausgegangen. Katalysator 14 repräsentiert den Stand der Technik, d. h. einen Kalium, Vanadin und Wolfram als Promotoren enthaltenden und Aluminium als Stabilisator enthaltenden Kontakt.

Nachstehend ist die Herstellung der erfindungsgemäßen Katalysatoren 1 bis 13 und des Vergleichskatalysators 14 beschrieben.

Katalysatoren 1 und 2 :

Die Katalysatoren werden hergestellt, indem Eisenrot mit einer Reinheit $> 99\%$ $\alpha$-$Fe_2O_3$ und die Promotoren auf eine Feinheit $< 20$ $\mu$m gemahlen und trocken vermischt werden. Dann wird das Pulvergemisch mit einer wäßrigen Lösung von KOH 3 h lang geknetet und anschließend zu Stängen extrudiert. Das Extrudat wird 2 h bei 110 °C getrocknet und anschließend 30 min bei 900 °C geglüht (Zusammensetzung vgl. Tabelle 1).

Katalysatoren 3-14 :

Die Katalysatoren werden hergestellt, indem gelbes Eisenoxidhydrat mit einer Reinheit $> 99\%$ FeOOH und die Promotoren auf eine Feinheit $< 20$ $\mu$m gemahlen und trocken vermischt werden. Dann wird das Pulvergemisch mit einer wäßrigen Lösung von KOH oder Kaliumcarbonat 3 h lang geknetet und anschließend zu Strängen extrudiert. Das Extrudat wird 2 h bei 110 °C und 1 h bei 300 °C getrocknet. Danach wird 30 min bei 950 °C geglüht (Zusammensetzung vgl. Tabelle 2).

Zur Feststellung der Aktivität, Selektivität und des Alkaliaustrags der Katalysatoren 1 bis 14 wurden diese einem Vergleichstest unterzogen, bei dem Ethylbenzol zu Styrol dehydriert wurde. Die Katalysatoren 1 bis 14 wurden auf eine Körnung von 0,5 bis 1 mm zerkleinert ; 20 ml der zerkleinerten Proben wurden jeweils in einen Reaktor mit einem Innendurchmesser von 6 mm gefüllt. Der Reaktor wurde durch ein Salzbad auf einer solchen Temperatur gehalten, daß die organische Phase am Reaktoraustrag 50 Gew.% Styrol enthielt. Der Versuch wurde 7 Tage lang durchgeführt. Die Ergebnisse der Versuche mit den Katalysatoren 1 bis 14 sind in der Tabelle 3 aufgeführt. Ein Vergleich der Versuchsergebnisse ergibt, daß die erfindungsgemäßen Katalysatoren bei vergleichbarer Selektivität und Aktivität einen wesentlich niedrigeren Kaliumaustrag aufweisen. Wegen der Kürze der Versuchsdauer ist bei den genannten Versuchsbedingungen noch keine Abnahme der Selektivität des Katalysators vom Stand der Technik zu beobachten.

(Siehe Tabellen Seite 5 ff.)

4

Tabelle 1

| Kataly-sator | Kalium Gehalt [% $K_2O$] | Einsatz-stoff | Element der V. Nebengruppe Gehalt [% $M_2O_5$] | | Element der VI. Nebengruppe Gehalt [% $MeO_3$] | |
|---|---|---|---|---|---|---|
| | | | Gehalt [% $M_2O_5$] | Einsatzstoff | Gehalt [% $MeO_3$] | Einsatzstoff |
| 1 | 11,8 | $K_2CO_3$ | 0,9 | $V_2O_5$ | 2,7 | $WO_3$ |
| 2 | 11,8 | $K_2CO_3$ | 1,4 | $V_2O_5$ | 1,1 | $MoO_3$ |

Tabelle 2

| Kataly-sator | Kalium Gehalt [% $K_2O$] | Einsatz-stoff | Element der V. Nebengruppe Gehalt [% $M_2O_5$] | Einsatzstoff | Element der VI. Nebengruppe Gehalt [% $MeO_3$] | Einsatzstoff |
|---|---|---|---|---|---|---|
| 3 | 11,8 | KOH | 0,9 | $V_2O_5$ | 2,7 | $WO_3$ |
| 4 | 11,8 | KOH | 0,6 | $NH_4VO_3$ | 3,2 | $MoO_3$ |
| 5 | 11,8 | KOH | 0,9 | $(VO)_3(PO_4)_2$ | 1,4 | $WO_3$ |
| 6 | 11,8 | KOH | 1,4 | $V_2O_5$ | 2,7 | $H_3[P(W_3O_{10})_4]$ |
| 7 | 11,8 | $K_2CO_3$ | 0,9 | $V_2O_5$ | 2,7 | $WO_3$ |
| 8 | 11,8 | KOH | 0,9 | $V_2O_5$ | 2,7 | $WO_3$ |
| 9 | 11,8 | KOH | 0,9 | $V_2O_5$ | 2,7 | $WO_3$ |
| 10 | 11,8 | KOH | 0,9 | $V_2O_5$ | 2,7 | $WO_3$ |
| 11 | 11,8 | KOH | 0,9 | $V_2O_5$ | 2,7 | $WO_3$ |
| 12 | 11,8 | KOH | 0,9 | $V_2O_5$ | 2,7 | $WO_3$ |
| 13 | 11,8 | KOH | 0,9 | $V_2O_5$ | 2,7 | $WO_3$ |
| Zum Vergleich | | | | | | |
| 14 | 11,8 | KOH | 0,9 | $V_2O_5$ | 2,7 | $WO_3$ |

0 072 439

0 072 439

Tabelle 1 (Fortsetzung)

| Kataly-sator | Element der V. Hauptgruppe | | Stabilisator | | Lithium | |
|---|---|---|---|---|---|---|
| | Gehalt $[\% \ Me_2O_5]$ | Einsatzstoff | Gehalt $[\% \ Me_2O_3]$ | Einsatzstoff | Gehalt $[\% \ Li_2O]$ | Einsatzstoff |
| 1 | – | – | 0,8 | $CrO_3$ | 0,3 | $Li_2CO_3$ |
| 2 | – | – | 1,5 | $AlO(OH)$ | 0,5 | $Li_3PO_4$ |

Tabelle 2

| Kataly-sator | Gehalt $[\% \ Me_2O_5]$ | Einsatzstoff | Gehalt $[\% \ Me_2O_3]$ | Einsatzstoff | Gehalt $[\% \ Li_2O]$ | Einsatzstoff |
|---|---|---|---|---|---|---|
| 3 | – | – | 1,5 | $AlO(OH)$ | 0,3 | $Li_2CO_3$ |
| 4 | – | – | 1,5 | $AlO(OH)$ | 0,3 | $Li_2CO_3$ |
| 5 | 1,3 | $(VO_3)(PO_4)_2$ | 1,5 | $AlO(OH)$ | 0,3 | $Li_2CO_3$ |
| 6 | 0,07 | $H_3\left[P(W_3O_{10})_4\right]$ | 1,5 | $AlO(OH)$ | 0,3 | $Li_2CO_3$ |
| 7 | 0,8 | $H_3PO_4$ | 1,5 | $AlO(OH)$ | 0,5 | $Li_2CO_3$ |
| 8 | – | – | 1,5 | $AlO(OH)$ | 0,15 | $Li_2CO_3$ |
| 9 | – | – | 1,5 | $AlO(OH)$ | 0,45 | $Li_2CO_3$ |
| 10 | – | – | 1,5 | $AlO(OH)$ | 0,6 | $Li_2CO_3$ |
| 11 | – | – | 1,5 | $AlO(OH)$ | 0,9 | $Li_2CO_3$ |
| 12 | 1,35 | $Li_3PO_4$ | 1,5 | $AlO(OH)$ | 0,3 | $Li_3PO_4$ |
| 13 | – | – | 1,5 | $AlO(OH)$ | 0,3 | $LiOH$ |
| Zum Vergleich | | | | | | |
| 14 | – | – | 1,5 | $AlO(OH)$ | – | – |

# 0 072 439

Tabelle 3

| Katalysator | Aktivität[x] [°C] | Selektivität [%] | Alkalisaustrag [%] |
|---|---|---|---|
| 1 | 601 | 96,1 | 2 |
| 2 | 604 | 96,3 | 1 |
| 3 | 598 | 96,4 | 0 |
| 4 | 600 | 95,9 | 1 |
| 5 | 606 | 96,4 | 0 |
| 6 | 601 | 96,6 | 0 |
| 7 | 607 | 96,3 | 1 |
| 8 | 601 | 95,8 | 5 |
| 9 | 602 | 96,3 | 0 |
| 10 | 600 | 96,3 | 0 |
| 11 | 603 | 96,1 | 0 |
| 12 | 605 | 96,7 | 1 |
| 13 | 599 | 96,0 | 6 |
| 14 | 602 | 96,4 | 21 |

[x]) Temperatur bei der 50 % Styrol in der organischen Phase des Reaktoraustrags enthalten sind.

## Patentansprüche

1. Dehydrierungskatalysator, enthaltend, bezogen auf a) bis e),
   a) 50 bis 95 Gew.% mindestens einer Eisenverbindung, berechnet als $Fe_2O_3$,
   b) 3 bis 50 Gew.% mindestens einer Kaliumverbindung, berechnet als $K_2O$,
   c) 0,1 bis 20 Gew.% einer Chrom- oder Aluminiumverbindung, berechnet als $Me_2O_3$, und
   d) 0,1 bis 20 Gew.% mindestens einer Verbindung eines Elementes der sechsten Nebengruppe des Periodensystems, berechnet als $MeO_3$ und/oder
   e) 0,1 bis 20 Gew.% mindestens einer Verbindung des Vanadins oder des Phosphors, berechnet als $Me_2O_5$, enthaltend ggf.
   f) weitere Zusätze,
dadurch gekennzeichnet, daß der Katalysator zusätzlich 0,1 bis 5 Gew.% einer Lithiumverbindung, berechnet als $Li_2O$, aufweist.

2. Verfahren zur Herstellung des Katalysators gemäß Patentanspruch 1, wobei aus Verbindungen der Komponenten a) bis e), ggf. unter Zugabe von f) und einer Lithiumverbindung, mit Wasser ggf. unter Zusatz von Verformungshilfsmitteln und/oder die Porosität verbessernden Mitteln eine Mischung hergestellt wird, die man knetet und verformt und wobei man die Formlinge zunächst bei Temperaturen von 80 bis 300 °C trocknet und danach bei Temperaturen von 700 bis 1 000 °C calciniert.

3. Verwendung von Katalysatoren nach Anspruch 1 zur Dehydrierung von Ethylbenzol.

## Claims

1. A dehydrogenation catalyst containing, based on a) to e),
   a) 50 to 95 % by weight of at least one iron compound, calculated as $Fe_2O_3$,
   b) 3 to 50 % by weight of at least one potassium compound, calculated as $K_2O$,
   c) 0.1 to 20 % by weight of a chromium or aluminium compound, calculated as $Me_2O_3$, and
   d) 0.1 to 20 % by weight of at least one compound of an element of group VIB of the periodic table, calculated as $MeO_3$, and/or

7

**0 072 439**

e) 0.1 to 20 % by weight of at least one vanadium or phosphorus compound, calculated as $Me_2O_5$, and optionally containing

f) further additives,

wherein the catalyst additionally contains from 0.1 to 5 % by weight of a lithium compound, calculated as $Li_2O$.

2. A process for the production of a catalyst as claimed in claim 1, wherein a mixture is prepared from compounds a) to e) and, if desired, f) ; a lithium compound ; water ; and optional shaping assistants and/or porosity-improving agents, which mixture is kneaded and shaped, and the resulting shaped articles are calcined first at 80 to 300 °C and then at 700 to 1 000 °C.

3. The use of a catalyst as claimed in claim 1 for dehydrogenating ethylbenzene.


**Revendications**

1. Catalyseur de déshydrogénation, contenant :

a) 50 à 95 % en poids d'au moins un composé du fer, calculé comme $Fe_2O_3$ ;

b) 3 à 50 % en poids d'au moins un composé du potassium, calculé comme $K_2O$ ;

c) 0,1 à 20 % en poids d'un composé du chrome ou de l'aluminium, calculé comme $Me_2O_3$ ;

d) 0,1 à 20 % en poids d'au moins un composé d'un élément du 6e sous-groupe du Tableau périodique, calculé comme $MeO_3$ ; et(ou)

e) 0,1 à 20 % en poids d'au moins un composé du vanadium ou du phosphore, calculé comme $Me_2O_5$, les pourcentages en poids se rapportant à la somme de a) à e), ainsi que

f) d'éventuels autres additifs,

caractérisé en ce que le catalyseur contient en outre entre 0,1 et 5 % en poids d'un composé du lithium calculé comme $Li_2O$.

2. Procédé de préparation d'un catalyseur selon la revendication 1, consistant à préparer un mélange des composants a) à e), du composant f) éventuel et d'un composé du lithium dans de l'eau, à y incorporer éventuellement des adjuvants de formage et(ou) des agents améliorant la porosité, à pétrir ce mélange et à le former en éléments façonnés, qui sont d'abord séchés entre 80 et 300 °C, puis calcinés entre 700 et 1 000 °C.

3. Utilisation de catalyseurs selon la revendication 1 pour la déshydrogénation de l'éthyl-benzène.